(19) Europäisches Patentamt
European Patent Office
Office européen des brevets

(11) Veröffentlichungsnummer: **0 294 746 A1**

# (12) EUROPÄISCHE PATENTANMELDUNG

(21) Anmeldenummer: **88109007.0**

(22) Anmeldetag: **06.06.88**

(51) Int. Cl.4: **C07C 79/10 , C07C 76/02 , C11B 9/00**

(30) Priorität: **12.06.87 CH 2205/87**

(43) Veröffentlichungstag der Anmeldung: **14.12.88 Patentblatt 88/50**

(84) Benannte Vertragsstaaten: **CH DE ES FR GB IT LI NL**

(71) Anmelder: **L. GIVAUDAN & CIE Société Anonyme**

**CH-1214 Vernier-Genève(CH)**

(72) Erfinder: **Bachmann, Jean-Pierre**
**Seestrasse 28**
**CH-8806 Bäch(CH)**
Erfinder: **Pesaro, Mario, Dr.**
**Wiesliacher 55**
**CH-8053 Zürich(CH)**
Erfinder: **Hochstetler, Alan R., Dr.**
**157 Rutland Road Glen Rock**
**N.J. 07452(US)**

(74) Vertreter: **Urech, Peter, Dr. et al**
**Grenzacherstrasse 124 Postfach 3255**
**CH-4002 Basel(CH)**

## (54) Bicyclische Nitrokörper.

(57) Die Erfindung betrifft neue Riechstoffe. Es handelt sich dabei um die Verbindungen der formel

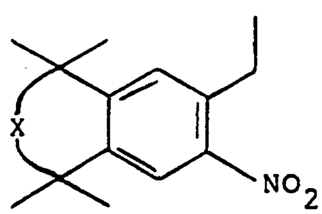

I

worin X für

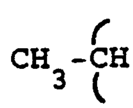 oder 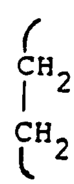

steht.

Die Formel I beinhaltet also das 5-Nitro-6-äthyl-1,1,2,3,3-pentamethylindan (Ia) und das 6-Nitro-7-äthyl-

EP 0 294 746 A1

1,1,4,4-tetramethyl-1,2,3,4-tetrahydro-naphthalin (Ib).

## Bicyclische Nitrokörper

Die Erfindung betrifft neue Riechstofe. Es handelt sich dabei um die Verbindungen der Formel

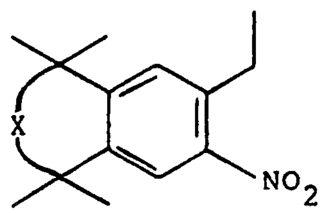

I

worin X für

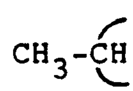 oder 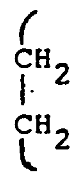

steht.

Die Formel I beinhaltet also das 5-Nitro-6-äthyl-1,1,2,3,3-pentamethylindan (Ia) und das 6-Nitro-7-äthyl-1,1,4,4-tetramethyl-1,2,3,4-tetrahydro-naphthalin (Ib).

Die Erfindung betrifft ferner ein Verfahren zur Herstellung der Verbindungen I.

Dieses Verfahren ist dadurch gekennzeichnet, dass man eine Verbindung der Formel

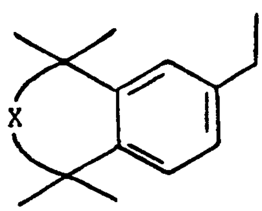

II

worin X obige Bedeutung besitzt, nitriert.

Die Nitrierung der Verbindungen der Formel II kann in an sich bekannter Weise mit einem Gemisch von ca. 40 bis 100%iger Salpetersäure und konz. Schwefelsäure bewerkstelligt werden (siehe z.B. Organikum, Organisch-chemisches Grundpraktikum, 13. Auflage, VEB Deutscher Verlag der Wissenschaften, Berlin 1974: S. 332 seq., und zwar dort insbesondere die Angaben betreffend den Aromaten "mittlerer Reaktivität).

Es hat sich zudem im vorliegenden Fall als zweckmässig erwiesen, die Verbindung II in einem organischen Lösungsmittel, beispielsweise einem halogenierten Kohlenwasserstoff wie Dichlormethan, Dichloräthan, etc. oder aber auch in Essigsäure gelöst, vorzulegen.

Der zweckmässige Temperaturbereich ist derjenige von ca. 0°C bis ca. 30°C und hängt insbesondere von der Konzentration der Salpetersäure und der Menge der konz. Schwefelsäure ab.

Zur Aufarbeitung wird das Reaktionsgemisch zweckmässigerweise entweder a) auf genügend Wasser gegossen und die auskristallisierten Nitrokörper abgenutscht, oder b) in ein mit Wasser nicht mischbaren Lösungsmittel aufgenommen, die Lösung neutral gewaschen, und das Lösungsmittel entfernt. Die so isolierten Nitrokörper können durch Umkristallisieren gereinigt werden.

Die Verbindungen der Formel I zeichnen sich durch kräftige, diffusive und sehr natürlich-warme Noten in Richtung Moschus, mit süssen und pudrigen Geruchsaspekten aus.

Die Erfindung betrifft demgemäss auch die Verwendung der Verbindungen I als Riechstoffe.

Die Verbindungen der Formel I eignen sich aufgrund ihrer natürlichen Geruchsnoten insbesondere zur Modifizierung von bekannten Kompositionen. Hervorgehoben werden soll insbesondere ihre ausserordentliche Geruchsstärke: Der Geruchsschwellenwert beträgt 0,29 bzw. 0,57 ng/l, der Geruchswert 2983 bzw. 2139. Bezüglich Definition von Geruchswert und Geruchsschwellenwert siehe z.B. Ulrich A. Huber, Seifen -

Oele - Fette - Wachse 110, Nr. 15 (1984) 448-451.

Die Verbindungen I verbinden sich mit zahlreichen bekannten Riechstoffingredientien natürlichen oder synthetischen Ursprungs, wobei die Palette der natürlichen Rohstoffe sowohl leicht- als auch mittel- und -schwer-flüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl, Paraguay, Wermutöl,
- Alkohole, wie Farnesol, Geraniol, Linalool, Nerol, Phenyläthylalkohol, Rhodinol, Zimtalkohol,
- Aldehyde, wie Citral, Helional®, α-Hexylzimtaldehyd, Hydroxycitronellal, Lilial® (p-tert.Butyl-α-methyl-dihydrozimtaldehyd), Methylnonylacetaldehyd,
- Ketone, wie Allyljonon, α-Jonon, β-Jonon, Isoraldein (Isomethyl-α-ionon), Methyljonon,
- Ester, wie Allyl-phenoxyacetat, Benzyl-salicylat, Cinnamylpropionat, Citronellyl-acetat, Citronellyl-äthoxolat (Citronellyl . O - CO -CO . $OC_2H_5$) Decyl-acetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat,
- Lactone, wie γ-Undecalacton,
- verschiedene, in der Parfümerie oft benützte Komponenten, wie Ketonmoschus, Indol, p-Methan-8-thiol-3-on, Methyleugenol.

Bemerkenswert ist ferner die Art und Weise, wie die Verbindungen I die Geruchsnoten bekannter Kompositionen abrunden und harmonisieren, ohne aber in unangenehmer Weise zu dominieren.

Und bemerkenswert ist schliesslich die ausgezeichnete Löslichkeit der Verbindungen I, wodurch sich applikatorische Vorteile ergeben.

Die Verbindungen der Formel I (bzw. deren Gemische) lassen sich in weiten Grenzen einsetzen, die beispielsweise von 0,1 (Detergentien) - 20% (alkoholische Lösungen) in Kompositionen reichen können, ohne das diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 1 und ca. 10%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I können demgemäss bei der Herstellung von Kompositionen und - wie obige Zusammenstellung zeigt - unter Verwendung einer breiten Palette bekannter Riechstoffe bzw. Riechstoffgemische, verwendet werden. Bei der Herstellung solcher Kompositionen können die oben aufgeführtne bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

## Beispiel 1

### 6-Nitro-7-äthyl-1,1,4,4-tetramethyl-1,2,3,4-tetrahydronaphthalin

10.8 g 7-Aethyl-1,1,4,4-tetramethyl-1,2,3,4-tetrahydronaphthalin werden in 10 ml Dichlormethan gelöst und während 70 Minuten bei 3-5 °C mit einem Gemisch von 9,3 ml 96%iger Schwefelsäure und 7,7 ml 65%iger Salpetersäure versetzt. Nach 6 Stunden wird auf Eis gegossen und mit Kochsalzlösung neutral gewaschen. Nach Trocknung und Einengen des Lösungsmittels verbleiben 12,7 g Kristalle, welche aus Hexan umkristallisiert werden. Man erhält so 6,63 g 6-Nitro-7-äthyl-1,1,4,4-tetramethyl-1,2,3,4-tetrahydronaphthalin vom Smp. 51-53 °C.

IR ($CHCl_3$): 1510, 1340 $cm^{-1}$;

$^1$H-NMR (400 MHz, $CDCl_3$): δ (ppm) 1,27 (t,3H); 1,30 (s,12H); 1,70 (s,4H); 2,89 (q,2H); 7,23 (s,1H); 7,87 (s,1H); MS (m/e): 261 ($M^+$), 246, 244, 216, 204, 190, 171, 158, 144, 128, 115, 105

Geruch: Moschus-artig, süss, pudrig.

Schwellenwert: 0,57 ng/l,

Geruchswert: 2'139.

Beispiel 2

5-Nitro-6-äthyl-1,1,2,3,3-pentamethylindan

48,0 g Aluminiumchlorid in 150 ml 1,2-Dichloräthan werden bei 20-25° C während 1,5 Stunden mit 56,4 g 1,1,2,3,3-Pentamethylindan und 25,9 g Acetylchlorid versetzt. Nach 1,5 Stunden wird auf Eis und konz. Salzsäure gegossen und mit Kochsalzlösung neutral gewaschen. Nach Einengen des Lösungsmittels verbleiben 80,5 g Rohprodukt. Nach der Destillation (Sdp. 102° C bei 0,05 mbar) erhält man 48,6 g 5-Acetyl-1,1,2,3,3-pentamethylindan.
IR ($CHCl_3$): 1675, 1600, 1570 $cm^{-1}$,
$^1$H-NMR (400 MHz, $CDCl_3$): $\delta$ (ppm) 1,01 (d,3H); 1,08 (s,3H); 1,09 (s,3H); 1,29 (s,3H); 1,32 (s,3H); 1,90 (q,1H); 2,59, (s,3H); 7,23 (d,1H); 7,78 (s,1H); 7,82 (d,1H);
MS (m/e): 230 ($M^+$), 215, 201, 185, 173, 159, 141, 128, 115, 105

30 g 5-Acetyl-1,1,2,3,3-pentamethylindan werden in 150 ml Aethanol gelöst und mit 3,0 g 5% Palladium/Kohle bei Raumtemperatur und Normaldruck hydriert. Man erhält so 28,0 g Rohprodukt, welche nach Destillation (Sdp. 72° C bei 0,18 mbar) 27,5 g 5-Aethyl-1,1,2,3,3-pentamethylindan ergeben.
$^1$H-NMR (400 MHz, $CDCl_3$): $\delta$ (ppm) 0,99 (d,3H); 1,06 (s,3H); 1,07 (s,3H); 1,27 (s,3H); 1,28 (s,3H); 1,25 (t,3H); 1,87 (q,1H); 2,65 (q,2H); 6,98 (s,1H); 7,04 (d,1H); 7,08 (d,1H);
MS (M/e): 216 ($M^+$), 201, 187, 173, 159, 145, 131, 117, 105

10,8 g 5-Aethyl-1,1,2,3,3-pentamethylindan werden in 10 ml Dichlormethan gelöst und während 45 Minuten bei einer Temperatur von 3-5° C mit einem Gemisch von 9,3 ml 96%iger Schwefelsäure und 7,7 ml 65%iger Salpetersäure versetzt. Nach 7 Stunden wird auf Eis gegossen und mit Kochsalzlösung neutral gewaschen. Nach Trocknung und Einengen des Lösungsmittels verbleiben 13,3 g Kristalle, welche aus Hexan umkristallisiert werden. Man erhält so 5,5 g 5-Nitro-6-äthyl-1,1,2,3,3-pentamethylindan vom Smp. 63-66° C.
IR ($CHCl_3$): 1515, 1350 $cm^{-1}$,
$^1$H-NMR (400 MHz, $CDCl_3$): $\delta$ (ppm) 1,02 (d,3H); 1,09 (s,6H); 1,28 (t,3H); 1,29 (s,6H); 1,90 (q,1H); 2,90 (q,2H); 7,07 (s,1H); 7,67 (s,1H);
MS (m/e): 261 ($M^+$), 246, 244, 232, 216, 200, 185, 174, 158, 144, 129, 115, 105
Geruch: moschus-artig,
Schwellenwert: 0,29 ng/l,
Geruchswert: 2'983.

Beispiel 3

A. Komposition mit blumigen, holzigen, moschus-artigen Noten

| | Gewichtsteile |
|---|---|
| Undecylenaldehyd | 1 |
| Bergamotteöl | 50 |
| Lilial | 100 |
| Heolione® [Firmenich] (Methyldihydrojasmonat) | 60 |
| Citronellol | 30 |
| Geraniol | 50 |
| Phenyläthylalkohol | 90 |
| Cetonial® [Givaudan] (3-(3,4-Methylendioxy-phenyl)-2-methyl-propional) | 20 |
| Rosenoxid | 1 |
| α-Hexylzimtaldehyd | 100 |
| Isoraldeine 70® [Givaudan] | 100 |
| Lyral® [IFF] (4,4-Methyl-4-hydroxyamyl-3-cyclohexen-carboxaldehyd) | 30 |
| p-tert.Butylcyclohexylacetat | 40 |
| Sandalore® [Givaudan] (3-Methyl-5-(2,2,3-trimethylcyclopent-3-en-1-yl)pental-2-ol) | 40 |
| Evernyl® [Roure Bertrand] (Methyl-ß-orcin-carboxylat) | 10 |
| Coumarin | 40 |
| γ-Undecalacton | 1 |
| Acetylcedrene | 120 |
| Verbindung Ia oder Ib | 100 |
| | 983 |

B. Komposition Fougère

| | Gewichtsteile |
|---|---|
| Lavendelöl | 210 |
| Amylsalicylat | 200 |
| Baum-moos absolue 50% in Dipropylenglykol | 100 |
| Citronellol | 100 |
| Geraniol | 80 |
| Bergamotteöl | 80 |
| α-Jonon | 80 |
| α-Amylzimtaldehyd | 25 |
| Eugenol | 20 |
| Methambrat® Givaudan (1-Acetoxy-1-methyl-2-sec.butyl-cyclohexan) | 25 |
| Verbindung Ia oder Ib | 50 |
| | 970 |

C. Komposition Chypre

| | Gewichtsteile |
|---|---|
| Aldehyd C-12-MNA 10% in Dipropylenglykol | 20 |
| Vetivenylacetat | 50 |
| Rhodinol | 50 |
| Patchouliöl | 50 |
| Baum-moos abs. 50% in Dipropylenglykol | 50 |
| Lilial | 100 |
| Hydroxycitronellal | 100 |
| Methyljonon | 100 |
| Coumarin | 100 |
| Bergamotteöl | 100 |
| Verbindung Ia oder Ib | 50 |
| | 770 |

D. Komposition Sandelholz

| | Gewichtsteile |
|---|---|
| Sandalore | 300 |
| Amyris-öl | 20 |
| Hydroxycitronellal | 30 |
| Methyljonon | 100 |
| Benzyl-benzoat | 400 |
| Verbindung Ia oder Ib | 30 |
| | 880 |

## Ansprüche

1. Verbindungen der Formel

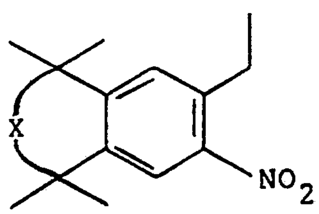

I

worin X für

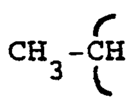 oder 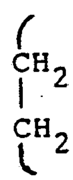

steht.

2. 5-Nitro-6-aethyl-1,1,2,3,3-pentamethylindan.

3. 6-Nitro-7-aethyl-1,1,4,4-tetramethyl-1,2,3,4-tetrahydronaphthalin.

4. Riechstoffkomposition, gekennzeichnet durch einen Gehalt an einer Verbindung der Formel

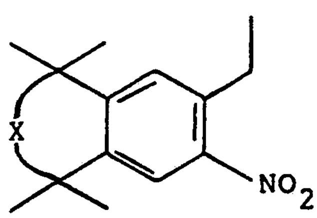

I

worin X für

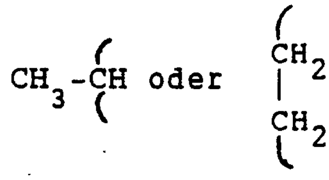

steht.

5. Verfahren zur Herstellung der Verbindungen der Formel

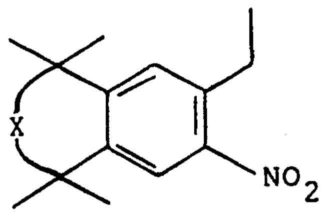

I

worin X für

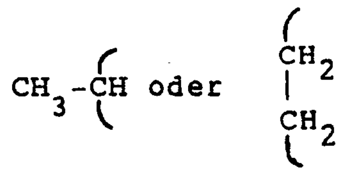

steht,
dadurch gekennzeichnet, dass man eine Verbindung der Formel

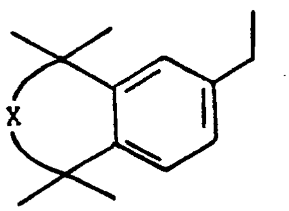

II

worin X obige Bedeutung besitst, nitriert.

6. Verwendung der verbindungen der Formel

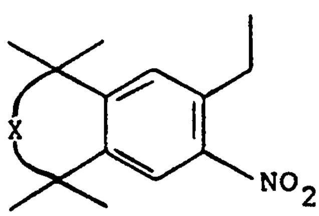

I

worin X für

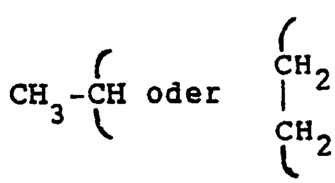

steht
als Riechstoff.

Europäisches Patentamt

# EUROPÄISCHER RECHERCHENBERICHT

Nummer der Anmeldung

EP 88 10 9007

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 652 400 (M.A. SPRECKER)<br>* Ansprüche; Beispiel II *<br>--- | 1-6 | C 07 C 79/10<br>C 07 C 76/02<br>C 11 B 9/00 |
| A | FR-A-1 456 956 (GIVAUDAN)<br>* Seite 1, Spalte 2, Zeile 4 von unten - Seite 2, Spalte 1, Zeile 1; Beispiel 20 *<br>----- | 1-6 | |
| | | | RECHERCHIERTE SACHGEBIETE (Int. Cl.4) |
| | | | C 07 C 79/00 |

Der vorliegende Recherchenbericht wurde für alle Patentansprüche erstellt

| Recherchenort | Abschlußdatum der Recherche | Prüfer |
|---|---|---|
| DEN HAAG | 01-09-1988 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)